# EUROPEAN PATENT APPLICATION

(11) **EP 4 699 604 A1**
(43) Date of publication of application: **25.02.2026**
(21) Application number: 24792150.5
(22) Date of filing: 19.04.2024
(51) Int. Cl.: A61K 9/72, C12N 15/861, C12N 7/01, A61P 37/04, A61K 35/761

(54) **INHALED ADENOVIRUS VECTOR VACCINE, PREPARATION METHOD THEREFOR AND USE THEREOF**

(30) Priority: 20.04.2023 CN 202310428062
(71) Applicant: Cansino Biologics Inc., Tianjin 300457 (CN)
(72) Inventor: ZHAO, Xiaolong, Tianjin 300457 (CN); PENG, Shaodan, Tianjin 300457 (CN); WANG, Liping, Tianjin 300457 (CN); WANG, Yueran, Tianjin 300457 (CN); JIAN, Hui, Tianjin 300457 (CN); JIANG, Yueying, Tianjin 300457 (CN); SI, Weixue, Tianjin 300457 (CN); XU, Fang, Tianjin 300457 (CN); ZHU, Tao, Tianjin 300457 (CN)
(74) Representative: Finnegan Europe LLP
(86) International application number: PCT/CN2024/088940
(87) International publication number: WO 2024/217567

(57) **Abstract**

The present invention relates to an inhaled aerosol vaccine, and in particular to a recombinant adenovirus vector inhaled aerosol vaccine, a preparation method therefor and a use thereof. The inhaled aerosol vaccine can simultaneously express one or more antigens. After inhaling and mucosal immunization are carried out on the body, a high-level specific antibody can be generated in the lung and the body at the same time, and a high cell immune response can be generated by means of stimulation. **In** addition, the vaccine is an inhaled preparation, which can effectively stimulate mucosal immunization and increase the third protection of the body. The recombinant adenovirus vector vaccine has good stability, is safe, efficient and painless, and can effectively cope with the recurrence of emerging infections and latent infections of bacteria and viruses.

## Description

### TECHNICAL FIELD

The present disclosure relates to the technical field of vaccines, particularly to an adenovirus inhalation vaccine, a preparation method therefor, and use thereof, and more particularly to a method for preparing a recombinant adenovirus vector vaccine for mucosal administration and use thereof.

### BACKGROUND

Since the 1920s, attempts have been made to administer drugs via the aerosol method to treat certain diseases. However, due to limitations in aerosol generation technology, inhalation therapy struggled to achieve the desired effect, making this route of administration a subject of ongoing debate. Currently, the mode of administration via nebulized aerosol inhalation is receiving increasing attention.

The columnar epithelial cells on the gastrointestinal tract, nasal cavity, and pulmonary tissue airways cannot be directly permeated in a vertical manner by the vast majority of polypeptides, proteins, and other macromolecular substances. However, the very tiny alveolar epithelial cells located deep in the pulmonary airways can allow certain macromolecular substances to pass through. Respiratory administration of polypeptides, proteins, and nucleic acids represents a rapidly developing new frontier in the research of drug delivery methods. Although aerosol generation technology is still in the developmental stage, the respiratory administration of polypeptides, proteins, and nucleic acids still has promising development prospects.

The superiority of respiratory aerosol immunization has made the method of respiratory aerosol immunization attract much attention from researchers today, mainly because it has several advantages over other immunization routes: convenience, high efficacy, non-invasiveness, and a reduced risk of cross-infection. Aerosol inhalation immunization is a promising immunization route, particularly suitable for children and the elderly. Compared with nasal instillation, it has unique advantages: it does not cause pain or inconvenience to patients, and thus is easily accepted by patients. Molecules that enter the lungs can not only induce a local mucosal immune response, but may even trigger an immune response in heterotopic mucosa.

It is already known that the lung tissue allows passage of macromolecular substances. However, fully elucidating the bioavailability of inhaled substances remains challenging, as the aerosol deposition rate and administration dose, the aerosol concentration, and the size distribution of particles (or droplets) in the nasopharynx and respiratory tract are all crucial factors. Therefore, there are still many problems with the treatment method of administering drugs via the respiratory tract.

Respiratory aerosol administration requires appropriate techniques. Studies have shown that the gastrointestinal tract, nasal cavity, and skin mucosa form robust barriers to the absorption of biological formulations.

Aerosol generation devices are in need of improvement, as rough equipment results in a lower drug concentration and also affects dose stability. Despite the large surface area of the deep respiratory tract (142 m² in adults) and its permeability characteristics, achieving an effective local drug concentration in the alveoli remains challenging. In fact, the low generation efficiency and lack of standardization of existing aerosol devices lead to variable dose stability among different patients. These are all issues demanding immediate solutions.

### SUMMARY

The present disclosure relates to an aerosol inhalation vaccine, particularly to a recombinant adenovirus vector aerosol inhalation vaccine, a preparation method therefor, and use thereof. The aerosol inhalation vaccine of the present disclosure can simultaneously express one or more antigens. After performing mucosal immunization on an organism via inhalation, the vaccine can not only induce high levels of specific antibodies in both the lungs and the body, but also stimulate a very strong cell-mediated immune response. Furthermore, the vaccine in the present disclosure is an inhalation formulation, which can effectively stimulate mucosal immunity and increase a third protection for the organism. The recombinant adenovirus vector vaccine provided by the present disclosure has good stability, is safe, efficient and painless, and can effectively cope with new infections caused by bacteria and viruses, as well as the recurrence of latent infections.

In one aspect of the present disclosure, provided is an aerosol inhalation formulation, which comprises an active ingredient, wherein the active ingredient is a recombinant adenovirus expressing an antigen protein, and the osmotic concentration of a solute molecule in the formulation is 300-750 mOsmol/kg; specifically, the osmotic concentration of the solute molecule in the formulation includes, but is not limited to, 300, 325, 350, 375, 400, 425, 450, 475, 500, 525, 550, 575, 600, 625, 650, 700, 725, or 750 mOsmol/kg.

Preferably, the osmotic concentration of the solute molecule in the formulation is 325-725 mOsmol/kg; specifically, the osmotic concentration of the solute molecule in the formulation includes, but is not limited to, 325, 350, 375, 400, 425, 450, 475, 500, 525, 550, 575, 600, 625, 650, 700, or 725 mOsmol/kg.

More preferably, the osmotic concentration of the solute molecule in the formulation is 375-625 mOsmol/kg; specifically, the osmotic concentration of the solute molecule in the formulation includes, but is not limited to, 375, 400, 425, 450, 475, 500, 525, 550, 575, 600, or 625 mOsmol/kg.

In some specific embodiments, the solute molecule includes a saccharide, a salt, a metal ion chelating agent, a protectant, and/or a surfactant.

In some specific embodiments, the content of the salt is 25-75 mM, and the salt is selected from at least one or two of sodium chloride, magnesium chloride, hydrohalide, sulfate, nitrate, phosphate, acetate, propionate, oxalate, malate, citrate, citrate, toluenesulfonate, benzoate, salicylate, and p-aminosalicylate.

In some specific embodiments, the saccharide is selected from at least one or two of sucrose, trehalose, fucose, and/or maltose.

In some specific embodiments, the content of the saccharide is 20-75 mg/mL; specifically, the content of the saccharide includes, but is not limited to, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, or 75 mg/mL.

In some specific embodiments, the protectant is selected from at least one or two of sorbitol, mannitol, erythritol, maltitol, lactitol, and xylitol.

In some specific embodiments, the content of the protectant is 20-75 mg/mL; specifically, the content of the protectant includes, but is not limited to, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, or 75 mg/mL.

In some specific embodiments, the sum of the contents of the saccharide, the salt, and the protectant is less than 500 mM.

In some specific embodiments, the surfactant is selected from one or more of Tween, Span, and glycerol; preferably, the surfactant is selected from at least one or two of Tween 20, Tween 80, Span 20, and Span 80.

In some specific embodiments, the concentration of the surfactant is 0.01-5.0 mg/mL; specifically, the concentration of the surfactant includes, but is not limited to, 0.02 mg/mL, 0.03 mg/mL, 0.04 mg/mL, 0.05 mg/mL, 0.06 mg/mL, 0.07 mg/mL, 0.08 mg/mL, 0.09 mg/mL, 0.1 mg/mL, 0.2 mg/mL, 0.3 mg/mL, 0.4 mg/mL, 0.5 mg/mL, 0.6 mg/mL, 0.7 mg/mL, 0.8 mg/mL, 0.9 mg/mL, 1 mg/mL, 1.5 mg/mL, 2 mg/mL, 2.5 mg/mL, 3 mg/mL, 3.5 mg/mL, 4.0 mg/mL, 4.5 mg/mL, or 5.0 mg/mL.

In some specific embodiments, the metal ion chelating agent is selected from: at least one or two of ethylenediaminetetraacetic acid (EDTA), ethylenediaminetetraacetic acid disodium salt (EDTA-2Na), nitrilotriacetic acid (also known as NTA), diethylenetriaminepentaacetic acid and salts thereof, citric acid (CA), tartaric acid (TA), and/or gluconic acid (GA).

In some specific embodiments, the formulation has a pH value of 1-14, specifically a pH value including but not limited to 1, 2, 3, 4, 5, 6, 7, 8, 9, 10, 11, 12, 13, or 14; preferably, the formulation has a pH value of 5-8, specifically a pH value including but not limited to 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, or 8.0; more preferably, the formulation has a pH value of 6-7.5, specifically a pH value including but not limited to 6.0, 6.1, 6.2, 6.3, 6.4, 6.5, 6.6, 6.7, 6.8, 6.9, 7.0, 7.1, 7.2, 7.3, 7.4, or 7.5.

In some specific embodiments, the formulation further comprises a buffer; preferably, the buffer is one or more of HEPES, HIS, TRIS, PB, succinic acid, and citric acid.

In some specific embodiments, the concentration of the buffer is 1-15 mM; specifically, the concentration of the buffer includes, but is not limited to, 1, 1.5, 2.0, 2.5, 3.0, 3.5, 4.0, 4.5, 5.0, 5.5, 6.0, 6.5, 7.0, 7.5, 8.0, 8.5, 9.0, 9.5, 10.0, 10.5, 11.0, 11.5, 12.0, 12.5, 13.0, 13.5, 14.0, 14.5, or 15.0 mM.

In some specific embodiments, the adenovirus is a human adenovirus or a chimpanzee adenovirus.

Preferably, the adenovirus is selected from one or more of human adenoviruses of serotypes 1 to 57.

In some specific embodiments, the chimpanzee adenovirus is selected from the group consisting of AdC8, AdC22, AdC30, AdC37, AdC11, AdC3, AdC17, AdC19, AdC31, AdC20, AdC24, Pan1, Pan2, Pan3, AdC16, AdC26, AdC82, AdC5, AdC7, AdC44, AdC38, AdC43, AdC63, AdC147, AdC73, AdC6, AdC55, AdC83, AdC143, AdC144, AdC145, AdC10, AdC28, AdC9, and AdC68; more preferably, the chimpanzee adenovirus is AdC6, AdC7, AdC63, or AdC68.

In some specific embodiments, the adenovirus is a replication-deficient recombinant adenovirus.

In some specific embodiments, the content of the recombinant adenovirus in the formulation is 1 × 10⁸ to 1 × 10¹² VP/mL; specifically, the content of the recombinant adenovirus in the formulation includes, but is not limited to, 1 × 10⁸ vp/mL, 2 × 10⁸ vp/mL, 3 × 10⁸ vp/mL, 4 × 10⁸ vp/mL, 5 × 10⁸ vp/mL, 6 × 10⁸ vp/mL, 7 × 10⁸ vp/mL, 8 × 10⁸ vp/mL, 9 × 10⁸ vp/mL, 1 × 10⁹ vp/mL, 2 × 10⁹ vp/mL, 3 × 10⁹ vp/mL, 4 × 10⁹ vp/mL, 5 × 10⁹ vp/mL, 6 × 10⁹ vp/mL, 7 × 10⁹ vp/mL, 8 × 10⁹ vp/mL, 9 × 10⁹ vp/mL, 1 × 10¹⁰ vp/mL, 2 × 10¹⁰ vp/mL, 3 × 10¹⁰ vp/mL, 4 × 10¹⁰ vp/mL, 5 × 10¹⁰ vp/mL, 6 × 10¹⁰ vp/mL, 7 × 10¹⁰ vp/mL, 8 × 10¹⁰ vp/mL, 9 × 10¹⁰ vp/mL, 1 × 10¹¹ vp/mL, 2 × 10¹¹ vp/mL, 3 × 10¹¹ vp/mL, 4 × 10¹¹ vp/mL, 5 × 10¹¹ vp/mL, 6 × 10¹¹ vp/mL, 7 × 10¹¹ vp/mL, 8 × 10¹¹ vp/mL, 9 × 10¹¹ vp/mL, or 1 × 10¹² vp/mL; preferably, the content of the recombinant adenovirus in the formulation is 5 × 10⁹ VP/mL.

In some specific embodiments, aerosol particles of the aerosol inhalation formulation have a D90 particle size of 5.0-10.0 µm; specifically, the particle size D90 includes, but is not limited to, 5.0, 5.5, 6.0, 6.5, 7.0, 7.5, 8.0, 8.5, 9.0, 9.5, or 10.0 µm.

Preferably, the D90 particle size is 6.0 µm, 6.1 µm, 6.2 µm, 6.3 µm, 6.4 µm, 6.5 µm, 6.6 µm, 6.7 µm, 6.8 µm, 6.9 µm, 7.0 µm, 7.1 µm, 7.2 µm, 7.3 µm, 7.4 µm, 7.5 µm, 7.6 µm, 7.7 µm, 7.8 µm, 7.9 µm, 8.0 µm, 8.1 µm, 8.2 µm, 8.3 µm, 8.4 µm, 8.5 µm, 8.6 µm, 8.7 µm, 8.8 µm, 8.9 µm, 9.0 µm, 9.1 µm, 9.2 µm, 9.3 µm, 9.4 µm, 9.5 µm, 9.6 µm, 9.7 µm, 9.8 µm, or 9.9 µm.

Specifically, the aerodynamic particle size MMAD is 1.5-6 µm, preferably 3.0-5.0 µm, and more preferably 3.1 µm, 3.2 µm, 3.3 µm, 3.4 µm, 3.5 µm, 3.6 µm, 3.7 µm, 3.8 µm, 3.9 µm, 4.0 µm, 4.1 µm, 4.2 µm, 4.3 µm, 4.4 µm, 4.5 µm, 4.6 µm, 4.7 µm, 4.8 µm, or 4.9 µm.

In some specific embodiments, aerosol particles of the aerosol inhalation formulation have a D50 particle size of 2.0-7.0 µm; specifically, the particle size D50 includes, but is not limited to, 2.0, 2.5, 3.0, 3.5, 4.0, 4.5, 5.0, 5.5, 6.0, 6.5, or 7.0 µm.

Preferably, the D50 particle size is 2.5-4 µm, preferably 2.6 µm, 2.7 µm, 2.8 µm, 2.9 µm, 3.0 µm, 3.1 µm, 3.2 µm, 3.3 µm, 3.4 µm, 3.5 µm, 3.6 µm, 3.7 µm, 3.8 µm, or 3.9 µm.

In some specific embodiments, aerosol particles of the aerosol inhalation formulation have a D10 particle size of 0.5-2.50 µm, specifically a D10 particle size including but not limited to 0.5, 1.0, 1.5, 2.0, or 2.5 µm; preferably, the D10 particle size is 0.7-1.5 µm, specifically including but not limited to 0.7, 0.8, 0.9, 1.0, 1.1, 1.2, 1.3, 1.4, or 1.5 µm.

In some specific embodiments, the NGI FPF (fine particle fraction) of the aerosol, representing the deposition percentage of particles smaller than 5.39 µm in the aerosol inhalation formulation, is not less than 45%.

Specifically, the NGI FPF (fine particle fraction) of the aerosol, representing the deposition percentage of particles smaller than 5.39 µm in the adenovirus vector vaccine inhalation solution, is not less than 45%, 50%, 55%, 60%, 65%, 70%, 75%, 80%, 85%, 90%, or 95%; preferably, the FPF is 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, or 85%.

Specifically, the NGI FPF (fine particle fraction) of the aerosol, representing the deposition percentage of particles smaller than 3.30 µm in the adenovirus vector vaccine inhalation solution, is not less than 15%, 20%, 25%, 30%, 35%, 40%, 45%, 46%, 47%, 48%, 49%, 50%, or 55%; preferably, the FPF is 25%, 30%, 35%, 40%, 45%, 46%, 47%, 48%, 49%, 50%, or 55%.

In some specific embodiments, the NGI FPF (fine particle fraction) of the aerosol, representing the deposition percentage of particles smaller than 3.30 µm in the aerosol inhalation formulation, is not less than 25%.

In some specific embodiments, the aerodynamic particle size MMAD is 1.5-6 µm, specifically including but not limited to 1.5, 2.0, 2.5, 3.0, 3.5, 4.0, 4.5, 5.0, 5.5, or 6.0 µm; preferably, the aerodynamic particle size MMAD is 3.0-5.0 µm, specifically including but not limited to 3.0, 3.1, 3.2, 3.3, 3.4, 3.5, 3.6, 3.7, 3.8, 3.9, 4.0, 4.1, 4.2, 4.3, 4.4, 4.5, 4.6, 4.7, 4.8, 4.9, or 5.0 µm.

In some specific embodiments, the antigen protein expressed by the recombinant adenovirus is derived from a viral or bacterial antigen, which is selected from: one or more of HIV, rabies virus, dengue virus, Ebola virus, coronavirus, human papillomavirus, hepatitis C virus, hepatitis B virus, rotavirus, measles virus, respiratory syncytial virus (RSV), varicella-zoster virus (VZV), cytomegalovirus, herpes simplex virus type 2, Epstein-Barr virus, human metapneumovirus, parainfluenza virus, influenza virus, *Trypanosoma cruzi* and *Plasmodium falciparum,* tubercle bacillus, *Streptococcus pneumoniae, Moraxella catarrhalis,* non-typeable *Haemophilus influenzae* (NTHi), *Neisseria meningitidis, Neisseria gonorrhoeae, Staphylococcus aureus, Helicobacter pylori, Mycobacterium tuberculosis,* and *Chlamydia trachomatis.* In another aspect of the present disclosure, provided is a method for preparing the formulation according to any one of the preceding embodiments, which comprises the following steps: preparing a recombinant adenovirus vector encoding a bacterial or viral antigen, and adding a pharmaceutically acceptable excipient.

In some specific embodiments, the method comprises the following steps: constructing a fusion antigen sequence; constructing a plasmid; and co-transfecting the obtained recombinant adenovirus shuttle plasmid and a backbone plasmid carrying most of the adenovirus genome into a host cell.

In some specific embodiments, the method further comprises the following steps: culturing the host cell; harvesting a replication-deficient recombinant adenovirus released from the host cell; subjecting the recombinant adenovirus to expansion culture; purifying the recombinant adenovirus obtained from the culture; and adding the purified recombinant adenovirus to a corresponding excipient to obtain the vaccine.

In another aspect of the present disclosure, provided is use of the aerosol inhalation formulation according to any one of the preceding embodiments or the method according to any one of the preceding embodiments in the preparation of a vaccine for preventing an infectious disease in a mammal.

In some specific embodiments, the vaccine is for immunization via mucosa.

Preferably, the mucosa includes oral mucosa or respiratory mucosa.

Preferably, the solvent is sterile water for injection.

Preferably, the unit dose of the formulation is 0.05-5 mL, preferably 0.1-1 mL.

Preferably, the content of the recombinant adenovirus in the formulation combination is 1 × 10⁸ to 10¹⁰ GC/dose, preferably 1 × 10⁸ to 2 × 10¹⁰ GC/dose.

Specifically, the vaccine is a mucosal immune formulation; the mucosal immune formulation is a nasal drop, an aerosol, a spray, a liquid formulation, a gel, a microsphere, a liposome, or a suspension.

Specifically, the mode of the mucosal administration is nasal inhalation or oral inhalation; preferably, the mode of the inhalation administration is inhalation of a liquid formulation.

Specifically, the unit dose of the vaccine is 0.05-0.5 mL.

Compared with the prior art, the present disclosure has the following beneficial effects: The adenovirus inhalation formulation of the present disclosure addresses the following issues: 1) subcutaneously injected vaccines and intramuscularly injected vaccines cannot induce sustained mucosal immunity and pulmonary immunity; 2) vaccines delivered via other methods cannot target pulmonary macrophages for immunization; 3) vaccines delivered via other methods cannot utilize the adenovirus vector as a safe, natural type I adjuvant to enhance vaccine immune responses in the lungs; and 4) these vaccines cannot suppress the recurrence of latent infections.

In one aspect of the present disclosure, the adenovirus inhalation formulation of the present disclosure is administered by respiratory mucosal delivery, which, compared with injection, not only offers compliance but also requires a lower dose, while additionally producing a triple immune effect involving humoral immunity, cellular immunity, and mucosal immunity. The vaccine composition provided by the present disclosure can be used for primary immunization or booster immunization.

In one aspect of the present disclosure, after being nebulized by a suitable device, the vaccine of the present disclosure can produce aerosol particles having a particle size of 2-10 µm, preferably 2.0-6 µm, with relatively good uniformity. By inhalation through the nasal cavity or oral cavity, the aerosol particles can reach the lungs, thereby producing a protective immune response for the entire respiratory tract and the lungs, enhancing the effective utilization rate of the vaccine, and improving the effect of the vaccine.

In one aspect of the present disclosure, the adenovirus inhalation formulation of the present disclosure is stable and can maintain viral activity for a relatively long period of time.

### BRIEF DESCRIPTION OF THE DRAWINGS

FIG. 1 shows the trend of IFU changes for Ad5 formulations with different osmotic pressures from Table 1 at 37 °C.
FIG. 2 shows the trend of IFU changes for Chadox1 formulations with different osmotic pressures from Table 2 at 37 °C.
FIG. 3 shows the trend of IFU changes for Ad26 formulations with different osmotic pressures from Table 2 at 37 °C.
FIG. 4 shows the trend of IFU changes for sAd36 formulations with different osmotic pressures from Table 2 at 37 °C.
FIG. 5 shows the effects of different saccharide/salt/protectant systems in adenovirus inhalation formulations from Table 3 on the trend of viral activity changes.

### DETAILED DESCRIPTION

The present disclosure is further described in detail below with reference to specific examples, and the examples are given only to illustrate the present disclosure, rather than limiting the scope of the present disclosure. The following examples are provided as a guide for further modification by those of ordinary skill in the art and are not intended to limit the present disclosure in any way.

Unless otherwise specified, the experimental methods in the following examples are all conventional methods, and are performed according to the techniques or conditions described in the literature in the art or according to the product instructions. The materials, reagents, and the like used in the following examples are all commercially available unless otherwise specified.

### Example 1: Recombinant Adenovirus Vaccine Packaging

### 1. Construction and preparation of fusion antigen strains

### (1) Construction of pDC316 plasmid

Synthesized GFP gene fragments were digested, and the digested fragments were recovered. Meanwhile, the shuttle plasmid vector of the AdMax adenovirus system was digested, and the vector was recovered. The fragments were separately ligated to the vectors using the homologous recombination method; and competent cells were transformed with the ligated vectors and spread on plates. Single clones were picked for streak inoculation, and meanwhile, for colony PCR identification. Then, single positive clones from the streaked plates were taken and cultured, and plasmids were extracted for enzyme digestion identification. Clones identified as positive by the enzyme digestion identification were taken for sequence determination.

### (2) Virus seed packaging of recombinant adenoviruses

Each shuttle plasmid and the backbone plasmid of the AdMax adenovirus system were co-transfected for packaging. Thus, Ad5, Chadox I, Ad26, and sAd36 viruses carrying the GFP gene were obtained.

### Example 2. Osmotic Pressure Study of Adenovirus Inhalation Formulations

An adenovirus inhalation formulation of Ad5 was formulated using histidine as a buffer. The osmotic concentration was adjusted by NaCl, and the osmotic concentration of each final formulation was measured. The effects of different osmotic concentrations on the stability of the adenovirus inhalation formulation (37 °C-3 days-1 week-2 weeks IFU) were studied, and the effects of different osmotic concentrations on the pulmonary response in mice were also studied. The specific groups are as follows:

**Table 1. Stability study of inhaled adenovirus (Ad5) formulations at different osmotic concentrations**

| Adenovirus | Group | Target osmotic concentration (mOsmol/L) |
|---|---|---|
| | 1 | 200±25 |
| | 2 | 300±25 |
| | 3 | 400±25 |
| | 4 | 500±25 |
| Ad5 | 5 | 600±25 |
| | 6 | 700±25 |
| | 7 | 800±25 |
| | 8 | 1000±25 |
| | 9 | 1200±25 |

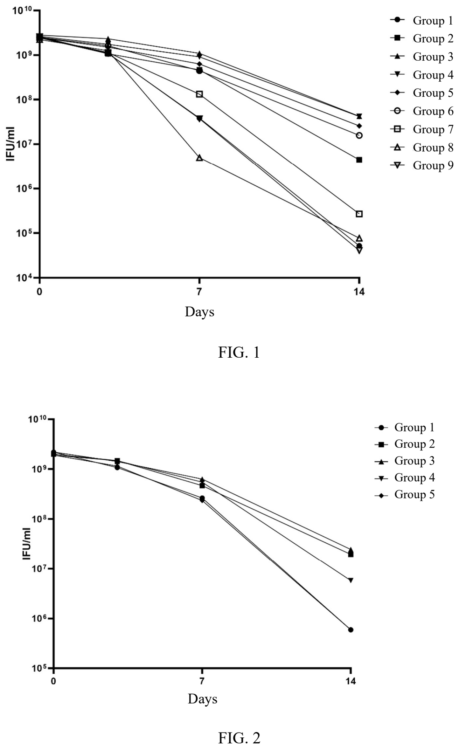

The results, as shown in FIG. 1, indicate that the adenovirus inhalation formulations in groups 3-6 exhibited relatively good stability, and the formulation stability was relatively good when the osmotic concentration was between 375-725 mOsmol/L.

### Example 3. Effect of Osmotic Pressure on Different Adenovirus Formulations

Adenovirus inhalation formulations of Chadox I, Ad26, and sAd36 were formulated using histidine as a buffer. The osmotic concentrations of the formulations were adjusted by NaCl to achieve final osmotic concentrations of 300-600 (±25) mOsmol/L. Then, the effect of osmotic pressure on different types of adenovirus formulations was tested. Details are as follows:

**Table 2. Stability study of inhaled adenovirus (Chadox I, Ad26, and sAd36) formulations at different osmotic concentrations**

| Adenovirus | Group | Target osmotic concentration (mOsmol/kg) |
|---|---|---|
| Chadox I | 1 | 300±20 |
| | 2 | 400±20 |
| | 3 | 500±20 |
| | 4 | 600±20 |
| | 5 | 700±20 |
| Ad26 | 6 | 300±20 |
| | 7 | 400±20 |
| | 8 | 500±20 |
| | 9 | 600±20 |
| | 10 | 700±20 |
| sAd36 | 11 | 300±20 |
| | 12 | 450±20 |
| | 13 | 550±20 |
| | 14 | 650±20 |
| | 15 | 750±20 |

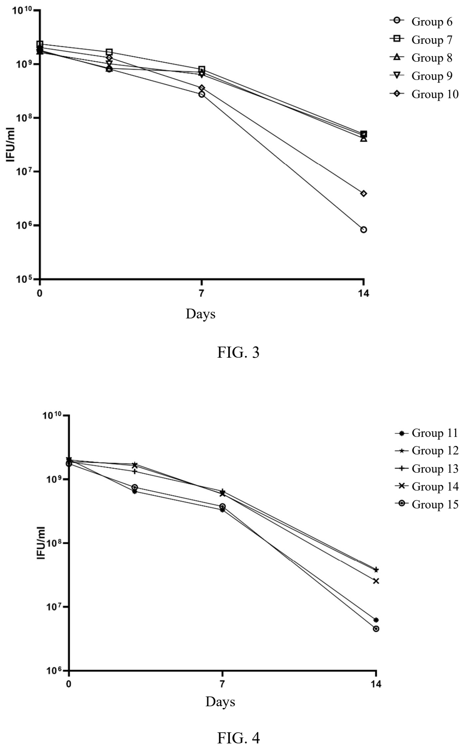

The results, as shown in FIGs. 2-4, indicate that the adenovirus inhalation formulations of Chadox I, Ad26, and sAd36 all maintained relatively good formulation stability when the osmotic concentration was between 375-725 mOsmol/L.

### Example 4. Effects of Different Saccharide/Salt/Protectant Systems on Viral Activity

An adenovirus inhalation formulation of Ad5 was formulated using histidine as a buffer. The osmotic concentration was adjusted by the salt, saccharide, and protectant contents. Then, the effects of different saccharide/salt/protectant systems on the adenovirus inhalation formulation were tested. Details are as follows:

**Table 3. Stability study of adenovirus inhalation formulations with different saccharide/salt/protectant systems in relation to osmotic concentration**

| Group | Sodium chloride | Sucrose | Trehalose | Mannitol | Sorbitol | Measured osmotic concentration |
|---|---|---|---|---|---|---|
| | mM | g/L | g/L | g/L | g/L | mOsmol/kg |
| 1 | 75 | 0 | | 100 | | 742 |
| 2 | 25 | 25 | | 100 | | 705 |
| 3 | 25 | / | 25 | 100 | / | 699 |
| 4 | 25 | 25 | / | | 100 | 711 |
| 5 | 25 | / | 25 | / | 100 | 701 |
| 6 | 25 | 0 | | 100 | | 662 |
| 7 | 75 | 75 | | 75 | | 752 |
| 8 | 75 | 0 | | 75g | | 612 |
| 9 | 25 | 25 | | 50 | | 459 |
| 10 | 25 | | 25 | 50 | | 463 |
| 11 | 25 | 25 | / | / | 50 | 449 |
| 12 | 25 | / | 25 | / | 50 | 453 |
| 13 | 50 | 50 | | 25 | | 411 |
| 14 | 75 | 75 | | 0 | | 413 |
| 15 | 50 | 50 | | 50 | | 585 |
| 16 | 25mM | 25g/L | | 25g/L | | 294 |

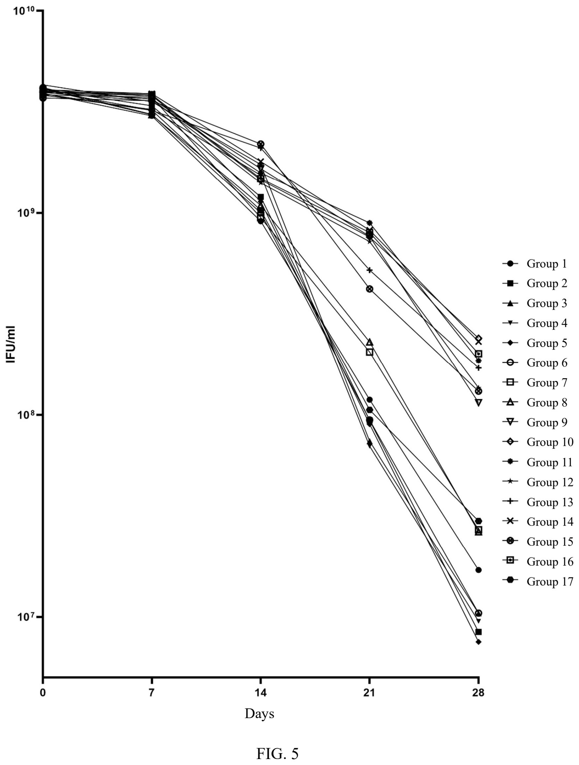

The results, as shown in FIG. 5, indicate that the formulations in groups 8-15 exhibited better stability.

### Example 5. Nebulized Particle Size Study of Adenovirus Inhalation Formulations

Based on the experimental results of Examples 2-4, the nebulization effects of the formulation systems were further investigated. The concentration and particle size distribution of the aerosol generated when using a nebulizer with different osmotic pressures were investigated. The aerosol particle size distribution of the vaccine after nebulization by the Aerogen Solo nebulizer was detected using an inhalation formulation particle size detector (Helos-Inhaler, Sympatec GmbH). The inhalation formulation particle size detector could detect in real time the parameters such as X10.3, X50.3, X90.3, VMD, optical concentration Copt, and Q3 ≤ 5.25 µm of the aerosol generated by atomization, where X10.3, X50.3, and X90.3 represent the cut-off particle sizes of 10%, 50%, and 90% portions in the cumulative volume distribution, respectively; the optical concentration Copt represents the concentration of the aerosol generated during the atomization process; VMD represents the volume mean diameter; and Q3 ≤ 5.25 µm represents the cumulative volume proportion of aerosol particles with a particle size less than 5.25 µm. Details are as follows:

**Table 4. Stability study of adenovirus inhalation formulations with different saccharide/salt/protectant systems in relation to osmotic concentration**

| Group | Sodium chloride | Sucrose | Mannitol | X_{10.3} | X_{50.3} | X_{90.3} | VMD | Q3≤5.25µm |
|---|---|---|---|---|---|---|---|---|
| 1 | 75 mM | 0 | 75 g/L | 0.92 | 2.81 | 7.85 | 3.72 | 77.21 |
| 2 | 25 mM | 25 g/L | 50 g/L | 0.89 | 2.82 | 8.32 | 3.84 | 76.08 |
| 3 | 50 mM | 50 g/L | 25 g/L | 0.85 | 2.76 | 8.04 | 3.72 | 77.32 |
| 4 | 75 mM | 75 g/L | 0 | 0.9 | 2.75 | 6.91 | 3.41 | 80.89 |
| 5 | 50 mM | 50 g/L | 50 g/L | 0.85 | 2.74 | 7.22 | 3.49 | 79.83 |

The preferred embodiments of the present disclosure are described in detail above, which, however, are not intended to limit the present disclosure. Within the scope of the technical concept of the present disclosure, various simple modifications can be made to the technical solutions of the present disclosure, all of which will fall within the protection scope of the present disclosure.

In addition, it should be noted that the various specific technical features described in the above specific embodiments can be combined in any suitable manner in which the features do not contradict each other. In order to avoid unnecessary repetition, various possible combinations will not be illustrated separately in the present disclosure.

## Claims

1. An aerosol inhalation formulation, comprising an active ingredient, wherein the active ingredient is a recombinant adenovirus expressing an antigen protein, and the osmotic concentration of a solute molecule in the formulation is 325-725 mOsmol/kg.

2. The aerosol inhalation formulation according to claim 1, wherein the osmotic concentration of the solute molecule in the formulation is 375-625 mOsmol/kg.

3. The aerosol inhalation formulation according to claims 1-2, wherein the solute molecule comprises a saccharide, a salt, a metal ion chelating agent, a protectant, and/or a surfactant.

4. The aerosol inhalation formulation according to claim 3, wherein the content of the salt is 25-75 mM, and the salt is selected from at least one or two of sodium chloride, magnesium chloride, hydrohalide, sulfate, nitrate, phosphate, acetate, propionate, oxalate, malate, citrate, toluenesulfonate, benzoate, salicylate, and p-aminosalicylate.

5. The aerosol inhalation formulation according to claim 3 or 4, wherein the content of the saccharide is 20-75 mg/mL, and the saccharide is selected from at least one or two of sucrose, trehalose, fucose, and/or maltose.

6. The aerosol inhalation formulation according to any one of claims 3-5, wherein the content of the protectant is 20-75 mg/mL, and the protectant is selected from at least one or two of sorbitol, mannitol, erythritol, maltitol, lactitol, and xylitol.

7. The aerosol inhalation formulation according to any one of claims 3-6, wherein the sum of the contents of the saccharide, the salt, and the protectant is less than 500 mM.

8. The aerosol inhalation formulation according to any one of claims 3-7, wherein the surfactant is selected from one or more of Tween, Span, and glycerol; preferably, the surfactant is selected from at least one or two of Tween 20, Tween 80, Span 20, and Span 80.

9. The aerosol inhalation formulation according to any one of claims 3-8, wherein the concentration of the surfactant is 0.01-5.0 mg/mL.

10. The aerosol inhalation formulation according to any one of claims 3-9, wherein the metal ion chelating agent is selected from: at least one or two of ethylenediaminetetraacetic acid (EDTA), ethylenediaminetetraacetic acid disodium salt (EDTA-2Na), nitrilotriacetic acid, diethylenetriaminepentaacetic acid and salts thereof, citric acid (CA), tartaric acid (TA), and/or gluconic acid (GA).

11. The aerosol inhalation formulation according to any one of claims 1-10, wherein the formulation has a pH value of 1-14; preferably, the formulation has a pH value of 5-8; more preferably, the formulation has a pH value of 6-7.5.

12. The aerosol inhalation formulation according to any one of claims 1-11, further comprising a buffer, wherein preferably, the buffer is one or more of HEPES, HIS, TRIS, PB, succinic acid, and citric acid; more preferably, the concentration of the buffer is 1-15 mM.

13. The aerosol inhalation formulation according to any one of claims 1-12, wherein the adenovirus is a human adenovirus or a chimpanzee adenovirus; preferably, the adenovirus is selected from one or more of human adenoviruses of serotypes 1 to 57.

14. The aerosol inhalation formulation according to claim 13, wherein the chimpanzee adenovirus is selected from the group consisting of AdC8, AdC22, AdC30, AdC37, AdC11, AdC3, AdC17, AdC19, AdC31, AdC20, AdC24, Pan1, Pan2, Pan3, AdC16, AdC26, AdC82, AdC5, AdC7, AdC44, AdC38, AdC43, AdC63, AdC147, AdC73, AdC6, AdC55, AdC83, AdC143, AdC144, AdC145, AdC10, AdC28, AdC9, and AdC68; more preferably, the chimpanzee adenovirus is AdC6, AdC7, AdC63, or AdC68.

15. The aerosol inhalation formulation according to any one of claims 1-14, wherein the adenovirus is a replication-deficient recombinant adenovirus.

16. The aerosol inhalation formulation according to any one of claims 1-15, wherein the content of the recombinant adenovirus in the formulation is 1 × 10⁸ to 1 × 10¹² VP/mL; preferably, the content of the recombinant adenovirus in the formulation is 5 × 10⁹ VP/mL.

17. The aerosol inhalation formulation according to any one of claims 1-16, wherein aerosol particles of the aerosol inhalation formulation have a D90 particle size of 5.0-10.0 µm.

18. The aerosol inhalation formulation according to any one of claims 1-16, wherein aerosol particles of the aerosol inhalation formulation have a D50 particle size of 2.0-7.0 µm.

19. The aerosol inhalation formulation according to any one of claims 1-16, wherein aerosol particles of the aerosol inhalation formulation have a D10 particle size of 0.5-2.50 µm, preferably 0.7-1.5 µm.

20. The aerosol inhalation formulation according to any one of claims 1-19, wherein the NGI FPF (fine particle fraction) of the aerosol, representing the deposition percentage of particles smaller than 5.39 µm in the aerosol inhalation formulation, is not less than 45%.

21. The aerosol inhalation formulation according to any one of claims 1-20, wherein the NGI FPF (fine particle fraction) of the aerosol, representing the deposition percentage of particles smaller than 3.30 µm in the aerosol inhalation formulation, is not less than 25%.

22. The aerosol inhalation formulation according to any one of claims 1-21, wherein the aerodynamic particle size MMAD is 1.5-6 µm, preferably 3.0-5.0 µm.

23. The aerosol inhalation formulation according to any one of claims 1-22, wherein the antigen protein expressed by the recombinant adenovirus is derived from a viral or bacterial antigen, which is selected from: one or more of HIV, rabies virus, dengue virus, Ebola virus, coronavirus, human papillomavirus, hepatitis C virus, hepatitis B virus, rotavirus, measles virus, respiratory syncytial virus (RSV), varicella-zoster virus (VZV), cytomegalovirus, herpes simplex virus type 2, Epstein-Barr virus, human metapneumovirus, parainfluenza virus, influenza virus, *Trypanosoma cruzi* and *Plasmodium falciparum,* tubercle bacillus, *Streptococcus pneumoniae, Moraxella catarrhalis,* non-typeable *Haemophilus influenzae* (NTHi), *Neisseria meningitidis, Neisseria gonorrhoeae, Staphylococcus aureus, Helicobacter pylori, Mycobacterium tuberculosis,* and *Chlamydia trachomatis.*

24. A method for preparing the aerosol inhalation formulation according to any one of claims 1-23, comprising the following steps: preparing a recombinant adenovirus vector encoding a bacterial or viral antigen, and adding a pharmaceutically acceptable excipient.

25. The method according to claim 24, comprising the following steps: constructing a fusion antigen sequence; constructing a plasmid; and co-transfecting the obtained recombinant adenovirus shuttle plasmid and a backbone plasmid carrying most of the adenovirus genome into a host cell.

26. The method according to claim 25, further comprising the following steps: culturing the host cell; harvesting a replication-deficient recombinant adenovirus released from the host cell; subjecting the recombinant adenovirus to expansion culture; purifying the recombinant adenovirus obtained from the culture; and adding the purified recombinant adenovirus to a corresponding excipient to obtain the vaccine.

27. Use of the aerosol inhalation formulation according to any one of claims 1-23 or the method according to claims 24-26 in the preparation of a vaccine for preventing an infectious disease in a mammal.

28. The use according to claim 27, wherein the vaccine is for immunization via mucosa; preferably, the mucosa comprises oral mucosa or respiratory mucosa.
